# EUROPEAN PATENT SPECIFICATION

(11) **EP 1 089 692 B1**
(45) Date of publication and mention of the grant of the patent: **02.05.2003**
(21) Application number: 99921064.4
(22) Date of filing: 01.06.1999
(51) Int. Cl.: A61F 13/15

(54) **DISPOSABLE ABSORBENT ARTICLE HAVING FECAL MANAGEMENT MEMBER**
ABSORBIERENDER WEGWERFARTIKEL MIT EINEM FÄKALIENMANAGEMENTELEMENT
ARTICLE ABSORBANT JETABLE A ELEMENT DE GESTION FECALE

(30) Priority: 04.06.1998 EP 98110154
(43) Date of publication of application: 11.04.2001
(73) Proprietor: THE PROCTER & GAMBLE COMPANY, Cincinnati, Ohio 45202 (US)
(72) Inventor: MÜLLER, Jörg, D-61184 Karben (DE); BAST, Tim, D-65824 Schwalbach (DE)
(74) Representative: McGregor, Judit Ester
(86) International application number: IB9900991
(87) International publication number: WO99062446

(56) References cited:
- WO-A-90/14061
- WO-A-91/01766
- WO-A-93/01780
- GB-A- 2 171 016
- GB-A- 2 294 397
- GB-A- 2 294 901
- US-A- 3 665 921

## Description

### FIELD OF THE INVENTION

The present invention relates to articles which absorb and/or contain bodily exudates, including disposable absorbent articles such as diapers, adult incontinence products, sanitary napkins and the like. More particularly, the invention relates to disposable absorbent articles which have the capacity to retain low-viscosity fecal material away from the skin of the wearer, in order to reduce leakage and make it easier to clean the wearer when the soiled disposable absorbent article is removed.

### BACKGROUND OF THE INVENTION

Disposable absorbent articles such as diapers and adult incontinence product are well known in the art. Such disposable absorbent articles collect and retain urine and fecal material deposited thereon by the wearer.

To date, most attempts in the art to handle the urine and fecal material collected and retained in the disposable absorbent article have been directed to handling urine. Dealing with fecal material collected by the disposable absorbent article is simply more difficult than dealing with urine, due to the complex rheology of low-viscosity material.

Exemplary of the urine handling prior art are several attempts to provide disposable absorbent articles having a first topsheet which faces towards and contacts the body of the wearer, and a secondary topsheet under the first topsheet, which either absorbs urine, or transfers the urine to an underlying core for storage until the disposable absorbent article is removed from the wearer.

Typically, the first topsheet and secondary topsheet have different material properties. The secondary topsheet may have a smaller pore size than the first topsheet, to assist in transfer of the urine through the topsheet. The first topsheet may be hydrophobic and more resilient when wetted than the secondary topsheet, in order to pass fluids through the first topsheet to the secondary topsheet.

In yet another attempt in the prior art, the disposable absorbent article has a first topsheet, secondary topsheet and core. The secondary topsheet consists essentially of meltblown hydrophilic fibers and has a pore size greater than the pore size of the core. This arrangement allegedly allows the secondary topsheet to rapidly receive multiple liquid insults and distribute the liquid in the X-Y plane prior to absorption by the core. In yet another attempt, improved vertical wicking capability for urine has been allegedly achieved using inflated cellulose fibers which are free of a surface finish or are crosslinked to one another.

In another attempt in the prior art, an absorbent core suitable for acquiring and containing liquids such as urine in a particularly effective and efficient manner comprises multiple layers. The first layer, which is closest to the wearer, comprises hydrophilic fibrous material and has an acquisition zone of a relatively lower average density than other portions of this layer, in order to quickly acquire discharged liquids. Below the first layer is a liquid handling layer comprising a resilient, low density high void volume material that is moisture insensitive in order to rapidly acquire liquid insults into itself through the acquisition zone and distribute these liquids throughout the liquid handling layer to a storage layer. The storage layer comprises a combination of fibrous material and discrete particles of absorbent gelling material, and allows the liquid handling layer to be drained of the liquids it has acquired, so that the liquid handling layer may have sufficient capacity to acquire and distribute subsequent loadings of liquids.

Examples of such attempts in the prior art include U.S. Patents 4,047,531 issued September 13, 1977 to Karami; 4,798,603 issued January 17, 1989 to Meyer et al.; 5,037,409 issued August 6, 1991 to Chen et al.; 5,124,197 issued June 23, 1992 to Bernardin et al.; and 5,134,007 issued July 28, 1992 to Reising et al.

Of course, absorbent gelling materials are also now well known in the prior art for their urine handling capability. Absorbent gelling materials are polymeric materials capable of absorbing large quantities of fluids, such as urine, and retaining such absorbed fluids under moderate pressure. The effectiveness of the absorbent gelling materials is quite dependent upon the form, position, and weight percentage of the absorbent gelling materials which are incorporated into the core of the disposable absorbent article.

Recent attempts have been made in the art to provide absorbent gelling materials which have the ability to swell against pressure. These teachings allege to provide the advantage that the absorbent gelling materials absorb fluid under actual pressures exerted by the body during use. Yet other teachings in the art provide absorbent gelling materials having a particular free swell rate and absorbency under load. Alleged advantages of such an absorbent gelling materials are lower volume and mass with approximately the same absorbent capacity, the ability to rapidly absorb a discharged liquid under pressures typically encountered during use, and the ability to retain the absorbed liquid under pressures typically encountered during use.

Examples of such attempts in the prior art include U.S. Patents 5,147,343 issued September 15, 1992 to Kellenberger and 5,149,335 issued September 22, 1992 to Kellenberger et al.

However, all of these attempts to handle urine do little, if anything, to improve handling of fecal material which may also be present in the disposable absorbent article. Attempts to deal with fecal material include providing a first topsheet which conforms closely to the wearer and has an aperture. The aperture is hopefully registered with the anal opening, so that fecal material passes therethrough into a void space. The first topsheet may comprise various elastic panels in order to closely conform to the skin of the wearer, and/or may have linear elastic strands. Improvements have been made in this area of the prior art, such as optimizing the material properties of the first topsheet. Such optimization makes the first topsheet more comfortable to the wearer and allows a single disposable absorbent article to fit a larger range of sizes of wearers.

Yet other attempts have been made in this area of the prior art to provide an absorbent core with a hole therein, in order to receive the fecal material. The hole may be oblate shaped, so that it is longer and narrower than the aperture in the first topsheet, or may be diamond shaped. The hole in the core may be positioned below an aperture which has elastic strips around its edge.

Improvements to this genre of the prior art disposable absorbent articles also include the addition of spacers. Spacers may be interposed between the first topsheet and the core, in order to ensure a void space is present to receive the fecal material.

Yet other attempts have been made in this genre of the prior art to provide barriers which limit the movement of fecal material to particular portions of the disposable absorbent article. The barriers limit the contact of the fecal material to a lesser portion of the skin of the wearer, than a comparable disposable absorbent article which has no barriers.

Still other attempts in the prior art provide barrier leg cuffs which are upstanding from the plane of the topsheet. The barrier leg cuffs prevent fecal material from breaching the perimeter of the disposable absorbent article.

Examples of such attempts to handle fecal material include U.S. Patent 4,892,536 issued January 9, 1990 to DesMarais et al.; U.S. Patent 4,909,803 issued March 20, 1990 to Aziz et al.; U.S. Patent 4,968,312 issued November 6, 1990 to Khan; commonly assigned U.S. Patent 4,990,147 issued February 5, 1991 to Freeland; commonly assigned U.S. Patent 5,037,416 issued August 6, 1991 to Allen et al.; U.S. Patent 5,062,840 issued November 5, 1991 to Holt et al.; commonly assigned U.S. Patent 5,171,236 issued December 15, 1992 to Dreier et al.; and European Patent Application 0,355,740 A2 published February 28, 1990 to Enloe.

However, none of these attempts to handle fecal material solve the problem of low-viscosity fecal material which is prevalent in younger children, particularly those who are breast fed. Low-viscosity fecal material easily migrates within the disposable absorbent article under the influences of gravity and motion or pressure by the wearer.

The migration of the fecal material often moves it towards the perimeter of the disposable absorbent article, increasing the likelihood of leakage. The migration of the fecal material also smears it against the skin of the wearer, making cleanup more difficult. In order to clean the wearer, the caretaker must wipe the entire area of the skin which has encountered the fecal material and typically has to deal with a relatively large soiled area.

One attempt in the art to handle low-viscosity fecal material is found in U.S. Patent Application Serial No. 08/076,713 filed June 11, 1993 in the name of Roe. This application is a disposable absorbent article having a first topsheet with a high trans-topsheet penetration overlaying a secondary topsheet having a lesser trans-topsheet penetration.

WO 93/01780 discloses an absorbent article comprising a support member and a layer of fibers.

Accordingly, it is an object of this invention to provide a diaper which reduces leakage of fecal material from the diaper and minimizes the amount of low-viscosity fecal material remaining on the skin of the wearer once the disposable absorbent article is removed. It is further an object of this invention to provide a diaper which separates the fecal material into components.

The object is solved by the features of claim 1.

Preferred embodiments an defined in the dependend claims.

### SUMMARY OF THE INVENTION

The present invention is directed to a (disposable) diaper. The disposable diaper comprises a liquid pervious topsheet, a backsheet joined to the topsheet, an absorbent core and a fecal management member positioned between the topsheet and the backsheet. The fecal management member comprises a support member and a plurality of fibers . This support member is a specific thin layer, as defined in claim 1. The fibers are woven into the support member have portions projecting from the support member. The fiber portions projecting from the support member may be of arcuate, loop or of pole form.

The absorbent core is positioned between the fecal management member and the backsheet.

### BRIEF DESCRIPTION OF THE DRAWINGS

While the specification concludes with claims particularly pointing out and distinctly claiming the subject matter which is regarded as the present invention, it is believed that the description will be better understood from the following descriptions which are taken in conjunction with the accompanying drawings in which like designations are used to designate substantially identical elements.
Figure 1 is a plan view of diaper of the present invention having portions cut away to reveal the underlying structure, the garment-facing surface of the diaper facing the viewer.
Figure 2 is a perspective illustration of a fecal management member of the present invention.
Figure 3 is a vertical cross-sectional illustration through a fecal management member of the present invention along a line connecting two points at which a fiber is woven into the support member.
Figure 4 is a vertical close-sectional illustration through a second fecal management member of the present invention along a line connecting two points at which a fiber is woven into the support member.
Figure 5 is a vertical cross-sectional illustration through a third fecal management member of the present invention along a line connecting two points at which a fiber is woven into the support member.
Figure 6 is a plan view illustration of another embodiment of a fecal management member of the present invention.
Figure 7 is a plan view illustration of another embodiment of a fecal management member of the present invention.

### DETAILED DESCRIPTION OF THE INVENTION

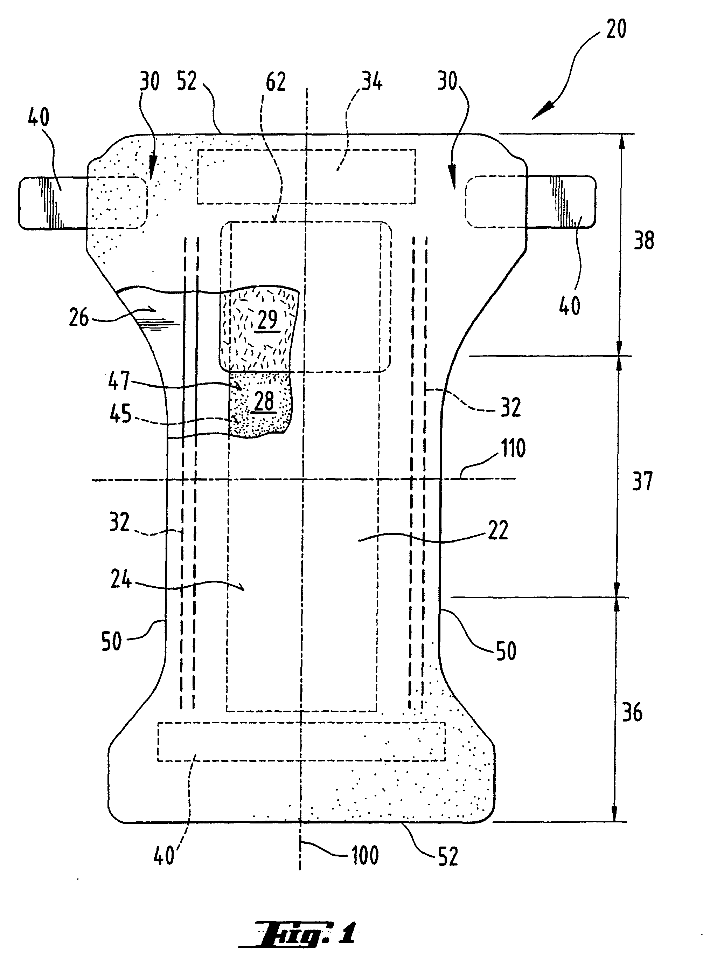

As used herein, the term "diaper" refers to a disposible absorbent article generally worn by infants and incontinent persons about the lower torso. When used herein, the term "absorbent article" refers to a diaper, as defined above. The term "disposable" is used herein to describe absorbent articles which generally are not intended to be laundered or otherwise restored or reused as an absorbent article (i.e., they are intended to be discarded after a single use and, preferably, to be recycled, composted or otherwise disposed of in an environmentally compatible manner). (As used herein, the term "disposed" is used to mean that an element(s) of the diaper is formed (joined and positioned) in a particular place or position as a unitary structure with other elements of the diaper or as a separate element joined to another element of the diaper. As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.) A "unitary" absorbent article refers to absorbent articles which are formed of separate parts united together to form a coordinated entity so that they do not require separate manipulative parts like a separate holder and liner. A preferred embodiment of an absorbent article of the present invention is the unitary disposable absorbent article, diaper 20, shown in Figure 1.

Figure 1 is a plan view of the diaper 20 of the present invention in a flat-out, state with portions of the structure being cut-away to more clearly show the construction of the diaper 20. The portion of the diaper 20 which faces the wearer is oriented towards the viewer. As shown in Figure 1, the diaper 20 preferably comprises a liquid pervious topsheet 24; a liquid impervious backsheet 26; an absorbent core 28, which is preferably positioned between at least a portion of the topsheet 24 and the backsheet 26; a fecal management member 29 positioned between the topsheet 24 and the absorbent core 28; side panels 30; elasticized leg cuffs 32; an elastic waist feature 34; and a fastening system generally designated 40. Diaper 20 is shown in Figure 1 to have a front waist region 36, a rear waist region 38 opposed to the front waist region 36 and a crotch region 37 located between the front waist region and the rear waist region. The periphery of the diaper 20 is defined by the outer edges of the diaper 20 in which the longitudinal edges 50 run generally parallel to the longitudinal centerline 100 of the diaper 20 and the end edges 52 run between the longitudinal edges 50 generally parallel to the lateral centerline 110 of the diaper 20.

The chassis 22 of the diaper 20 comprises the main body of the diaper 20. The chassis 22 comprises at least a portion of the absorbent core 28 and preferably an outer covering layer including the topsheet 24 and the backsheet 26. If the absorbent article comprises a separate holder and a liner, the chassis 22 generally comprises the holder and the liner. (For example, the holder may comprise one or more layers of material to form the outer cover of the article and the liner may comprise an absorbent assembly including a topsheet, a backsheet, and an absorbent core. In such cases, the holder and/or the liner may include a fastening element which is used to hold the liner in place throughout the time of use.) For unitary absorbent articles, the chassis 22 comprises the main structure of the diaper with other features added to form the composite diaper structure. While the topsheet 24, the backsheet 26, and the chassis 22 may be assembled in a variety of well known configurations, preferred diaper configurations are described generally in U.S. Patent 3,860,003 entitled "Contractible Side Portions for Disposable Diaper" which issued to Kenneth B. Buell on January 14, 1975; and U.S. Pat. No. 5,151,092 issued to Buell on September 9, 1992; and U.S. Pat. No. 5,221,274 issued to Buell on June 22, 1993. Other suitable diaper chassis design are disclosed in U.S. Pat. No. 5,569,232 entitled "Absorbent Article With Multiple Zone Structural Elastic-Like Film Web Extensible Waist Feature" which issued to Roe et al. on October 29, 1996; U.S. Pat. No. 5,554,144 entitled "Absorbent Article With Multiple Zone Structural Elastic-Like Film Web Extensible Waist Feature" which issued to Roe et al. on September 10, 1996; U.S. Pat. No. 5,554,143 entitled "Absorbent Article With Multiple Zone Structural Elastic-Like Film Web Extensible Waist Feature" which issued to Roe et al. on September 10, 1996; U.S. Pat. No. 5,554,145 entitled "Absorbent Article With Multiple Zone Structural Elastic-Like Film Web Extensible Waist Feature" which issued to Roe et al. on September 10, 1996; U.S. Pat. No. 5,556,394 entitled "Absorbent Article With Multiple Zone Structural Elastic-Like Film Web Extensible Waist Feature" which issued to Roe et al. on September 17, 1996.

The backsheet 26 is generally that portion of the diaper 20 positioned adjacent the garment facing surface 45 of the absorbent core 28 which prevents the exudates absorbed and contained therein from soiling articles which may contact the diaper 20, such as bedsheets and undergarments. In preferred embodiments, the backsheet 26 is impervious to liquids (e.g., urine) and comprises a thin plastic film such as a thermoplastic film having a thickness of about 0.012 mm (0.5 mil) to about 0.051 mm (2.0 mils). Suitable backsheet films include those manufactured by Tredegar Industries Inc. of Terre Haute, IN and sold under the trade names X15306, X10962 and X10964. Other suitable backsheet materials may include breathable materials which permit vapors to escape from the diaper 20 while still preventing exudates from passing through the backsheet 26. Exemplary breathable materials may include materials such as woven webs, nonwoven webs, composite materials such as film-coated nonwoven webs, and microporous films such as manufactured by Mitsui Toatsu Co., of Japan under the designation ESPOIR NO and by EXXON Chemical Co., of Bay City, TX, under the designation EXXAIRE. Suitable breathable composite materials comprising polymer blends are available from Clopay Corporation, Cincinnati, OH under the name HYTREL blend P18-3097. Such breathable composite materials are described in greater detail in PCT Application No. WO 95/16746, published on June 22, 1995 in the name of E. I. DuPont and copending U.S. Patent Application Serial No. 08/744,487, filed on November 6, 1996 in the name of Curro. Other breathable backsheets including nonwoven webs and apertured formed films are described in U.S. Pat. No. 5,571,096 issued to Dobrin et al. on November 5, 1996.

The backsheet 26, or any portion thereof, may be elastically extensible in one or more directions. In one embodiment, the backsheet 26 may comprise a structural elastic-like film ("SELF") web. A structural elastic-like film web is an extensible material that exhibits an elastic-like behavior in the direction of elongation without the use of added elastic materials. The SELF web includes a strainable network having at least two contiguous, distinct, and dissimilar regions. Preferably, one of the regions is configured so that it will exhibit resistive forces in response to an applied axial elongation in a direction parallel to the predetermined axis before a substantial portion of the other region develops significant resistive forces to the applied elongation. At least one of the regions has a surface-pathlength which is greater than that of the other region as measured substantially parallel to the predetermined axis while the material is in an untensioned condition. The region exhibiting the longer surface-pathlength includes one or more deformations which extend beyond the plane of the other region. The SELF web exhibits at least two significantly different stages of controlled resistive force to elongation along at least one predetermined axis when subjected to an applied elongation in a direction parallel to the predetermined axis. The SELF web exhibits first resistive forces to the applied elongation until the elongation of the web is sufficient to cause a substantial portion of the region having the longer surface-pathlength to enter the plane of applied elongation, whereupon the SELF web exhibits second resistive forces to further elongation. The total resistive forces to elongation are higher than the first resistive forces to elongation provided by the first region. SELF webs suitable for the present invention are more completely described in U.S. Patent No. 5,518,801 entitled Web Materials Exhibiting Elastic-Like Behavior, which issued to Chappell, et, al. on May 21, 1996, which is incorporated herein by reference. In alternate embodiments, the backsheet 26 may comprise elastomeric films, foams, strands, or combinations of these or other suitable materials with nonwovens or synthetic films.

The backsheet 26 may be joined to the topsheet 24, the absorbent core 28 or any other element of the diaper 20 by any attachment means known in the art. For example, the attachment means may include a uniform continuous layer of adhesive, a patterned layer of adhesive, or an array of separate lines, spirals, or spots of adhesive. One preferred attachment means comprises an open pattern network of filaments of adhesive as disclosed in U.S. Patent 4,573,986 entitled "Disposable Waste-Containment Garment", which issued to Minetola et al. on March 4, 1986. Other suitable attachment means include several lines of adhesive filaments which are swirled into a spiral pattern, as is illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. Each of these patents are incorporated herein by reference. Adhesives which have been found to be satisfactory are manufactured by H. B. Fuller Company of St. Paul, Minnesota and marketed as HL-1258. Alternatively, the attachment means may comprise heat bonds, pressure bonds, ultrasonic bonds, dynamic mechanical bonds, or any other suitable attachment means or combinations of these attachment means as are known in the art.

The topsheet 24 is preferably positioned adjacent the body surface 47 of the absorbent core 28 and may be joined thereto and/or to the backsheet 26 by any attachment means known in the art. Suitable attachment means are described above with respect to means for joining the backsheet 26 to other elements of the diaper 20. In one preferred embodiment of the present invention, the topsheet 24 and the backsheet 26 are joined directly to each other in some locations and are indirectly joined together in other locations by directly joining them to other elements of the diaper 20.

The topsheet 24 is preferably compliant, soft feeling, and non-irritating to the wearer's skin. Further, at least a portion of the topsheet 24 is liquid pervious, permitting liquids to readily penetrate through its thickness. A suitable topsheet 24 may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. If the absorbent assemblies include fibers, the fibers may be spunbond, carded, wet-laid, meltblown, hydroentangled, or otherwise processed as is known in the art. One suitable topsheet 24 comprising a web of staple length polypropylene fibers is manufactured by Veratec, Inc., a Division of International Paper Company, of Walpole, Massachusetts under the designation P-8.

Suitable formed film topsheets are described in U.S. Pat. No. 3,929,135, entitled "Absorptive Structures Having Tapered Capillaries", which issued to Thompson on December 30, 1975; U.S. Pat. No. 4,324,246 entitled "Disposable Absorbent Article Having A Stain Resistant Topsheet", which issued to Mullane, et al. on April 13, 1982; U.S. Patent 4,342,314 entitled "Resilient Plastic Web Exhibiting Fiber-Like Properties", which issued to Radel, et al. on August 3, 1982; U.S. Pat. No. 4,463,045 entitled "Macroscopically Expanded Three-Dimensional Plastic Web Exhibiting Non-Glossy Visible Surface and Cloth-Like Tactile Impression", which issued to Ahr, et al. on July 31, 1984; and U.S. Pat. No. 5,006,394 "Multilayer Polymeric Film" issued to Baird on April 9, 1991. Other suitable topsheets 30 are made in accordance with U.S. Pat. Nos. 4,609,518 and 4,629,643 which issued to Curro et al. on September 2, 1986 and December 16, 1986, respectively. Such formed films are available from The Procter & Gamble Company of Cincinnati, Ohio as "DRI-WEAVE" and from Tredegar Corporation of Terre Haute, Indiana as "CLIFF-T."

Preferably, the topsheet 24 is made of a hydrophobic material or is treated to be hydrophobic in order to isolate the wearer's skin from liquids contained in the absorbent core 28. If the topsheet 24 is made of a hydrophobic material, preferably at least the upper surface of the topsheet 24 is treated to be hydrophilic so that liquids will transfer through the topsheet more rapidly. This diminishes the likelihood that body exudates will flow off the topsheet 24 rather than being drawn through the topsheet 24 and being absorbed by the absorbent core 28. The topsheet 24 can be rendered hydrophilic by treating it with a surfactant or by incorporating a surfactant into the topsheet. Suitable methods for treating the topsheet 24 with a surfactant include spraying the topsheet 24 material with the surfactant and immersing the material into the surfactant. A more detailed discussion of such a treatment and hydrophilicity is contained in U.S. Pat. No. 4,988,344 entitled "Absorbent Articles with Multiple Layer Absorbent Layers" issued to Reising, et al. on Jan. 29, 1991 and U.S. Pat. No. 4,988,345 entitled "Absorbent Articles with Rapid Acquiring Absorbent Cores" issued to Reising on Jan. 29, 1991. A more detailed discussion of some suitable methods for incorporating surfactant in the topsheet can be found in U.S. Statutory Invention Registration No. H1670, published on July 1, 1997 in the names of Aziz et al. Each of these references is hereby incorporated by reference herein.

Any portion of the topsheet 24 may be coated with a lotion as is known in the art. Examples of suitable lotions include those described in U.S. Pat. No. 5,607,760 entitled "Disposable Absorbent Article Having A Lotioned Topsheet Containing an Emollient and a Polyol Polyester Immobilizing Agent" which issued to Roe on March 4, 1997; U.S. Pat. No. 5,609,587 entitled "Diaper Having A Lotioned Topsheet Comprising A Liquid Polyol Polyester Emollient And An Immobilizing Agent" which issued to Roe on March 11, 1997; U.S. Pat. No. 5,635,191 entitled "Diaper Having A Lotioned Topsheet Containing A Polysilozane Emollient" which issued to Roe et al. on June 3, 1997; and U.S. Pat. No. 5,643,588 entitled "Diaper Having A Lotioned Topsheet" which issued to Roe et al. on July 1, 1997. The topsheet may also include or be treated with antibacterial agents, some examples of which are disclosed in PCT Publication No. WO 95/24173 entitled "Absorbent Articles Containing Antibacterial Agents in the Topsheet For Odor Control" which was published on September 14, 1995 in the name of Johnson. Further, the topsheet 24, the backsheet 26 or any portion of the topsheet or backsheet may be embossed and/or matte finished to provide a more cloth like appearance.

The absorbent core 28 may comprise any absorbent material which is generally compressible, conformable, non-irritating to the wearer's skin, and capable of absorbing and retaining liquids such as urine and other certain body exudates. The absorbent core 28 may be manufactured in a wide variety of sizes and shapes (e.g., rectangular, hourglass, "T"-shaped, asymmetric, etc.) and may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The configuration and construction of the absorbent core 28 may also be varied (e.g., the absorbent core(s) or other absorbent structure(s) may have varying caliper zones, a hydrophilic gradient, a superabsorbent gradient, or lower average density and lower average basis weight acquisition zones; or may comprise one or more layers or structures). However, the total absorbent capacity of the absorbent core 28 should be compatible with the design loading and the intended use of the diaper 20.

Exemplary absorbent structures for use as the absorbent assemblies are described in U.S. Patent 4,610,678 entitled "High-Density Absorbent Structures" issued to Weisman et al. on September 9, 1986; U.S. Patent 4,673,402 entitled "Absorbent Articles With Dual-Layered Cores" issued to Weisman et al. on June 16, 1987; U.S. Patent 4,834,735, entitled "High Density Absorbent Members Having Lower Density and Lower Basis Weight Acquisition Zones", issued to Alemany et al. on May 30, 1989; U.S. Patent 4,888,231 entitled "Absorbent Core Having A Dusting Layer" issued to Angstadt on December 19, 1989; U.S. Pat. No. 5,137,537 entitled "Absorbent Structure Containing Individualized, Polycarboxylic Acid Crosslinked Wood Pulp Cellulose Fibers" which issued to Herron et al. on August 11, 1992; and U.S. Patent 5,147,345 entitled "High Efficiency Absorbent Articles For Incontinence Management" issued to Young et al. on September 15, 1992; U.S. Pat. No. 5,342,338 entitled "Disposable Absorbent Article For Low-Viscosity Fecal Material" issued to Roe on August 30, 1994.

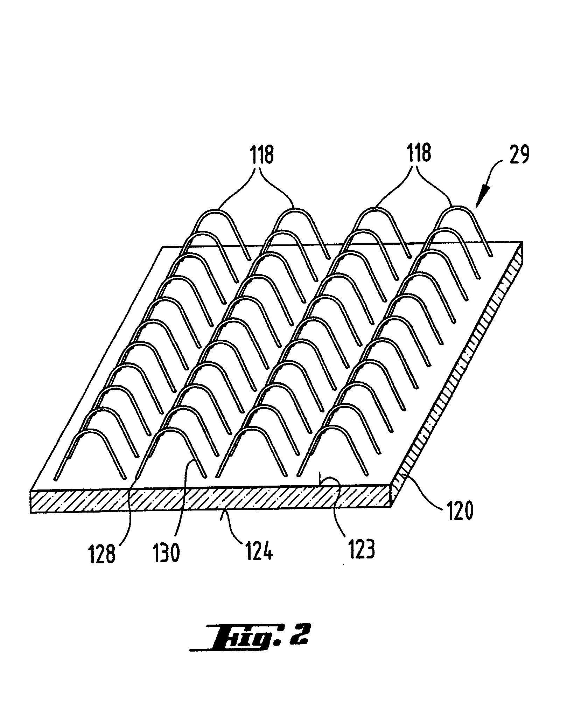

FIG. 2 illustrates an embodiment of the fecal management member according to the present invention, generally designated by the reference numeral 29.

The fecal management member 29 comprises a support member 120 and a plurality of fibers 118 which are woven into the support member 120 and which have portions 130 which are projecting from the support member 120.

The support member 120 comprises a front major surface 123 and a rear major surface 124. In the absorbent article according to the present invention, the fecal management member 29 is oriented such that the front surface 123 of the support member is facing towards the skin of the wearer. In one embodiment of the fecal management member of the present invention, the fibers project from both major surfaces of the support member. In other embodiments of the present invention, the fibers project only from one surface of the support member which can be either the front surface or the back surface.

In a preferred embodiment of the fecal management member 29 of the present invention, the fibers 118 are woven into the support member at regularly spaced weaving locations 128, the fibers being at least partially oriented parallel to each other. The weaving locations 128 preferably are continuous in at least one direction along the major surface of the support element 120 such that the weaving locations 128 form continuous lines along this direction. Preferably, the fibers 118 connect at least two weaving locations 128 which are spaced apart perpendicular to the above direction of continuous weaving locations. The continuous lines of weaving locations 128 may form regular patterns on the surface of the support member such that the projecting portions of the woven fibers exhibit the same regular patterns.

In another equally preferred embodiment of the present invention, the weaving locations are grouped together such that the woven fibers are arranged in a tufted configuration. In this case, a tuft projecting from the surface of the support member may comprise fiber ends and/or fibers connecting one tuft with each other. Preferably, the tufts are arranged in a regular pattern.

The portions of the fibers projecting from the support member can have different forms. Preferably, they are of arcuate form, loop form or of pole form.

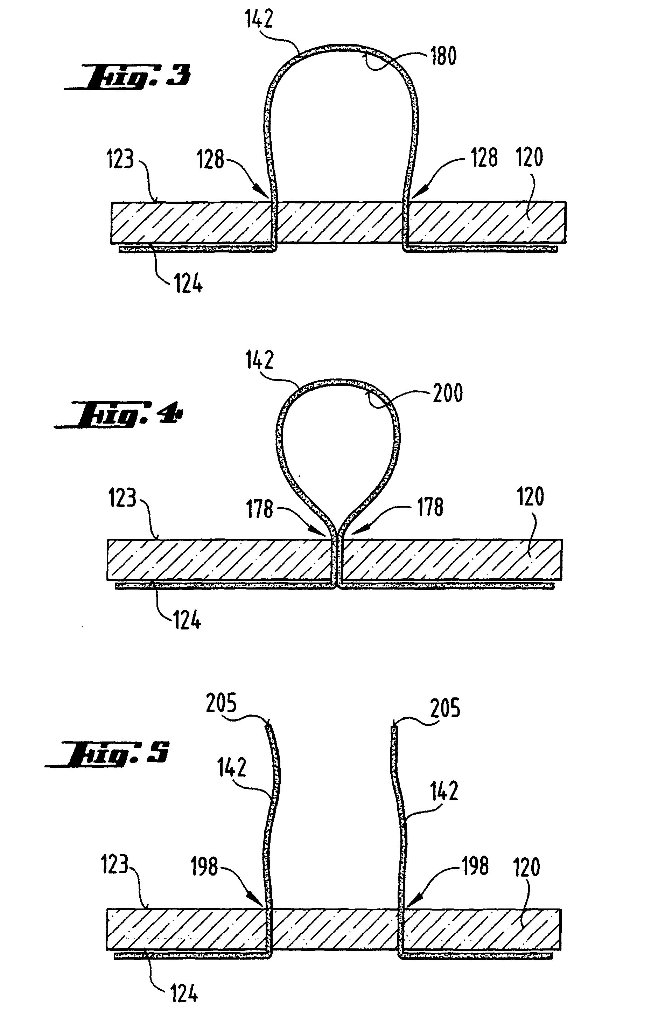

FIG. 3 illustrates a fiber portion 180 projecting from the support member 120 which connects two weaving locations 128 in an arcuate form. The two weaving locations 128 are space apart along the surface of the support member and the fiber 142 exhibits a curved configuration between the two weaving locations 128.

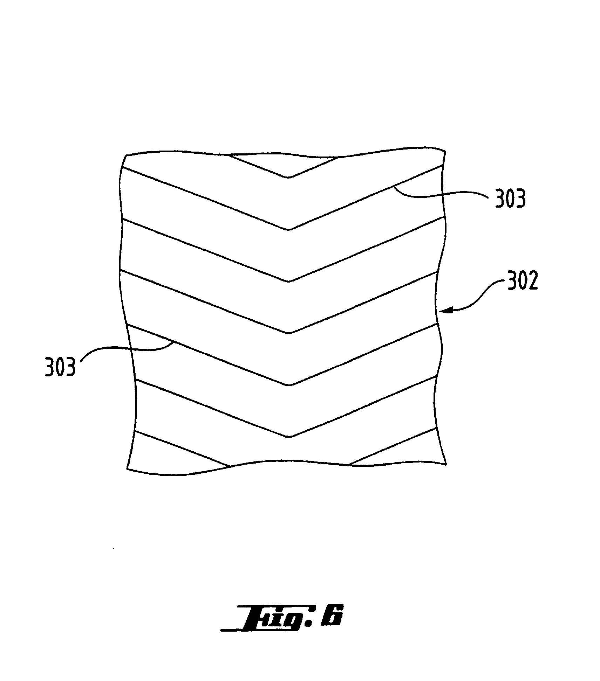

FIG. 4 shows a fiber portion 200 projecting from the support member 120 which connects two weaving locations 178 in a loop form. The two weaving locations 178 are in close proximity to each other, preferably less than two fiber diameters. The fiber 142 may also touch itself at the weaving locations 178. In particular, the two weaving locations may coincide. The fiber 142 exhibits a curved configuration between the two weaving locations.

FIG. 5 shows two fiber portions 205 projecting from the support member in pole form at two spaced apart weaving locations 198, the two portions 205 being fiber ends; the respective fibers 142 being woven into the support member 120.

The projecting fiber portions have a generally uniform height from the support member of greater than about 0.5 millimeters and preferably greater than about 1.0 millimeters, the height of the projecting fibers portions is at least one third, and preferably one half to one and one half times the distance between the weaving locations, the individual fibers are less than 300 denier (preferably in the range of 15 to 30 denier) in size, and the plurality of fibers without the support member has a basis weight in the range of 5 to 300 grams per square meter (and preferably in the range of 15 to 100 grams per square meter) measured along the first surface to provide sufficient open area between the fibers in the sheet of fibers along the projecting portions (i.e., between about 10 and 90 percent open area) to afford ready penetration of fecal material into the individual fibers along the projecting portions.

The support member is made of a material selected from limited to thermoplastic films, porous films, apertured films, apertured formed films, unapertured formed films, nonwoven webs, breathable materials, such as breathable films, including but not limited to microporous films, apertured nonwoven webs and the like. The support member is a relatively thin layer having a thickness in the range of about 0.00125 to 0.05 centimeters.

The fibers can be disposed in various directions with respect to the parallel weaving locations and may or may not be bonded together at crossover points in the projecting portions; can be disposed in various directions with respect to the parallel weaving locations with the majority of the fibers (i.e., over 80 or 90 percent) extending in directions at about a right angle to the weaving locations; or all of the individual fibers in the sheet of fibers can extend in directions generally at right angles to the spaced generally parallel weaving locations.

The fibers preferably have a hydrophilicity which is less than the hydrophilicity of the support member. In a preferred embodiment, the fibers themselves have a hydrophilicity gradient wherein the fiber portions projecting from the front major surface have a hydrophilicity which is less than the hydrophilicity of the fiber portions which are on the opposite side of the front major surface.

Preferably the fecal management member 29 is secured to the topsheet 24 in a very minimal extent to preserve the openness of the fecal management member 29 to allow ready penetration of fecal material. More preferably, the fecal management member 29 is not secured to the topsheet 24 at all preserving the openness of the fecal management member 29 and also allowing the topsheet 24 to separate from the fecal management member 29 creating additional void space within the disposable absorbent article. However, it is recognized that the fecal management member 29 should be secured within the diaper 20 to prevent it from freely moving about. To this end, it is preferred that the fecal management member 29 be secured directly to the underlying absorbent core 28. The fecal management member 29 may be joined to the absorbent core 28 by any attachment means known in the art. For example, the attachment means may include a uniform continuous layer of adhesive, a patterned layer of adhesive, or an array of separate lines, spirals, or spots of adhesive. One preferred attachment means comprises an open pattern network of filaments of adhesive as disclosed in U.S. Patent 4,573,986 entitled "Disposable Waste-Containment Garment", which issued to Minetola et al. on March 4, 1986. Other suitable attachment means include several lines of adhesive filaments which are swirled into a spiral pattern, as is illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. Adhesives which have been found to be satisfactory are manufactured by H. B. Fuller Company of St. Paul, Minnesota and marketed as HL-1258. Alternatively, the attachment means may comprise heat bonds, pressure bonds, ultrasonic bonds, dynamic mechanical bonds, or any other suitable attachment means or combinations of these attachment means as are known in the art. A particularly preferred attachment means is an adhesive having a hydrophilicity which is greater than the hydrophilicity of the sheet of fibers 126, more preferably a hydrophilicity which is also greater than the hydrophilicity of the support member 120.

The fecal management member 29 is shown in FIG. 1 to be positioned in the rear waist region 38 of the diaper 20. By positioning the fecal management member 29 at least in the rear waist region 38, the fecal management member 29 is aligned with the wearer's anus where it can be most effective in the management of fecal material deposited onto the diaper 20. However, the fecal management member 29 may extend into crotch region 37. The fecal management member 29 may also extend into the front waist region 36 of the diaper 20. In some embodiments it may also be desirable to have the fecal management member 29 extend along the entire longitudinal dimension of the diaper 20.

In addition to positioning the fecal management member 29 at least in the rear waist region 38 it is also desirable that the portion of the topsheet 24 positioned within the rear waist region 38 have a sufficient open area to handle low-viscosity fecal material.

To be the most effective in the handling of low-viscosity fecal material the fecal management member must have a lofted open structure. One key component of this equation is the height of the projecting portions of the fibers from the support member. As mentioned above the projecting portions of the fibers have a generally uniform height from the support member of greater than about 0.5 millimeters and preferably greater than about 1.0 millimeters. While even greater heights would provide excellent handling of low-viscosity fecal material, e.g., heights of 5.0 centimeters, such heights would create unwanted bulk in the diaper which may cause discomfort for the wearer.

Another key property of the fecal management member of the present invention is its resistance to compression under pressure. As used herein, the term " compression resistance" refers to the percentage caliper a fecal management member exhibits under a certain load compared to its caliper in the unloaded condition. In use, the fecal management member must be able, at least partially, to maintain its openness under the load of the wearer to be able to readily accept fecal material. Preferably, the fecal management member has a compression resistance of at least 30% under an applied pressure of 981N/cm², more preferably the fecal management member has a compression resistance of at least 40% under an applied pressure of 981N/cm², most preferably the fecal management member has a compression resistance of at least 50% under an applied pressure of 981N/cm².

Another key component is the resiliency of the fecal management member 29. As used herein, the term "resiliency" refers to the percentage recovered caliper after a fecal management member has been compressed under a certain load for a certain time. In order to remain open, the fecal management member must have a sufficient resiliency to withstand the forces of packaging and those applied by the wearer. This material property is measured using the Resiliency Test described in the present application. Preferably, the fecal management member has a resiliency of at least 50% after 30 seconds under an applied pressure of 981N/cm², more preferably, the fecal management member has a resiliency of at least 75% after 30 seconds under an applied pressure of 981N/cm², most preferably, the fecal management member has a resiliency of at least 85% after 30 seconds under an applied pressure of 981N/cm².

In another embodiment of the present invention, the fecal management member comprises a support structure which includes a second sheet of support member material. The second sheet of support member material is adhered on the side of the support member layer opposite the projecting fibers. The second sheet of support member material in the support member is a preferably a polymeric film. Other suitable materials for use as the second sheet of support member material include but are not limited to porous films, apertured films, apertured formed films, unapertured formed films, nonwoven webs, breathable materials, such as breathable films, including but not limited to microporous films, apertured nonwoven webs and the like. The second sheet of support member material is a relatively thin layer having a thickness in the range of about 0.00125 to 0.025 centimeters.

In another embodiment of the present invention, the fecal management member comprises a support member that includes a plurality of fibers. The fibers of the support member can be disposed in various directions with respect to the parallel weaving locations and may or may not be bonded together; they can be disposed in various directions with respect to the parallel weaving locations with the majority of the fibers in the support member (i.e., over 80 or 90 percent) extending in directions at about a right angle to the weaving locations; or all of the individual fibers in the support member can extend in directions generally at right angles to the spaced generally parallel weaving locations. Preferably, the individual fibers are not bonded together and are spaced from one another by a distance of from about 0.01 mm to about 10.0 mm. The fibers have a denier within the range of about 0 - 50.

FIG. 6 is a top plan view of another embodiment of a fecal management member 302 of the present invention. Fecal management member 302 depicts one of many possible configuration for the arrangement of the rows of projecting portions 303.

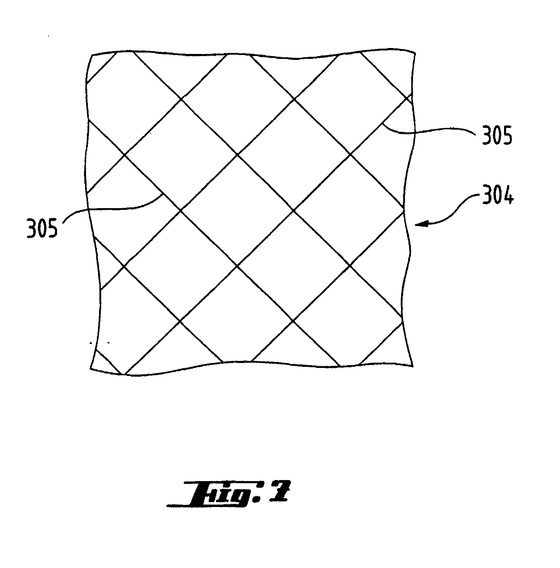

FIG. 7 is a top plan view of another embodiment of a fecal management member 304 of the present invention. Fecal management member 304 depicts one of many possible configuration for the arrangement of the rows of projecting portions 305.

In addition to the configurations shown in FIGS. 6 and 7, other possible configurations (e.g. with more intricate patterns) for the arrangement of the rows of projecting portions may also be used.

The diaper 20 may also comprise at least one elastic waist feature 34 that helps to provide improved fit and containment. The elastic waist feature 34 is generally intended to elastically expand and contract to dynamically fit the wearer's waist. The elastic waist feature 34 preferably extends at least longitudinally outwardly from at least one waist edge 62 of the absorbent core 28 and generally forms at least a portion of the end edge 52 of the diaper 20. Disposable diapers are often constructed so as to have two elastic waist features, one positioned in the first waist region 36 and one positioned in the second waist region 38. Further, while the elastic waist feature 34 or any of its constituent elements may comprise one or more separate elements affixed to the diaper 20, the elastic waist feature 34 may be constructed as an extension of other elements of the diaper 20, such as the backsheet 26, the topsheet 24, or both the backsheet 26 and the topsheet 24.

The elastic waist feature 34 may be constructed in a number of different configurations including those described in U.S. Patent 4,515,595 issued to Kievit et al. on May 7, 1985; U.S. Patent 4,710,189 issued to Lash on December 1, 1987; U.S. Pat. No 5, 151,092 issued to Bueli on September 9, 1992; and U.S. Pat. No. 5,221,274 issued to Buell on June 22, 1993. Other suitable waist configurations may include waistcap features such as those described in U.S. Patent 5,026,364 issued to Robertson on June 25, 1991 and U.S. Patent 4,816,025 issued to Foreman on March 28, 1989.

The diaper 20 may also include a fastening system 40. The fastening system 40 preferably maintains the first waist region 36 and the second waist region 38 in an overlapping configuration so as to provide lateral tensions about the circumference of the diaper 20 to hold the diaper 20 on the wearer. The fastening system 40 preferably comprises tape tabs and/or hook and loop fastening components, although any other known fastening means are generally acceptable. Some exemplary fastening systems are disclosed in U.S. Patent 3,848,594 entitled "Tape Fastening System for Disposable Diaper" issued to Buell on November 19, 1974; U.S. Patent B1 4,662,875 entitled "Absorbent Article" issued to Hirotsu et al. on May 5, 1987; U.S. Patent 4,846,815 entitled "Disposable Diaper Having An Improved Fastening Device" issued to Scripps on July 11, 1989; U.S. Patent 4,894,060 entitled "Disposable Diaper With Improved Hook Fastener Portion" issued to Nestegard on January 16, 1990; U.S. Patent 4,946,527 entitled "Pressure-Sensitive Adhesive Fastener And Method of Making Same" issued to Battrell on August 7, 1990; and the herein before referenced U.S. Pat. No. 5,151,092 issued to Buell on September 9, 1992; and U.S. Pat. No. 5, 221,274 issued to Buell on June 22, 1993. The fastening system may also provide a means for holding the article in a disposal configuration as disclosed in U.S. Pat. No. 4,963,140 issued to Robertson et al. on October 16, 1990.

The diaper 20 may also comprise side panels 30. The side panels 30 may be elastic or extensible to provide a more comfortable and contouring fit by initially conformably fitting the diaper 20 to the wearer and sustaining this fit throughout the time of wear well past when the diaper 20 has been loaded with exudates since the elasticized side panels 30 allow the sides of the diaper 20 to expand and contract. The side panels 30 may also provide more effective application of the diaper 20 because even if the diaperer pulls one elasticized side panel 30 farther than the other during application, the diaper 20 will "self-adjust" during wear.

While the diaper 20 of the present invention preferably has the side panels 30 disposed in the second waist region 38, the diaper 20 may be provided with side panels 30 disposed in the first waist region 36 or in both the first waist region 36 and the second waist region 38. The side panels 30 may be constructed in any suitable configurations. Examples of diapers with elasticized side panels are disclosed in U.S. Patent 4,857,067, entitled "Disposable Diaper Having Shirred Ears" issued to Wood, et al. on August 15, 1989; U.S. Patent 4,381,781 issued to Sciaraffa, et al. on May 3, 1983; U.S. Patent 4,938,753 issued to Van Gompel, et al. on July 3, 1990; the herein before referenced U.S. Pat. No. 5,151,092 issued to Buell on September 9, 1992; and U.S. Pat. No. 5, 221,274 issued to Buell on June 22, 1993; U.S. Patent No. 5,669,897 issued to LaVon, et al. on September 23, 1997 entitled "Absorbent Articles Providing Sustained Dynamic Fit"; U.S. Patent Application Serial No. 08/155,048 entitled "Absorbent Article With Multi-Directional Extensible Side Panels" filed November 19, 1993 in the names of Robles, et al.

The diaper 20 preferably further includes leg cuffs 32 which provide improved containment of liquids and other body exudates. Leg cuffs may also be referred to as leg bands, side flaps, barrier cuffs, or elastic cuffs. U.S. Patent 3,860,003 describes a disposable diaper which provides a contractible leg opening having a side flap and one or more elastic members to provide an elasticized leg cuff (a gasketing cuff). U.S. Patent Nos. 4,808,178 and 4,909,803 issued to Aziz et al. on February 28, 1989 and March 20, 1990, respectively, describe disposable diapers having "stand-up" elasticized flaps (barrier cuffs) which improve the containment of the leg regions. U.S. Patents 4,695,278 and 4,795,454 issued to Lawson on September 22, 1987 and to Dragoo on January 3, 1989, respectively, describe disposable diapers having dual cuffs, including gasketing cuffs and barrier cuffs.

The diaper 20 embodiment of the present invention may be applied to a wearer by positioning one of the waist regions, preferably the rear waist region 38, under the wearer's back and drawing the remainder of the diaper 20 between the wearer's legs. The other waist region, preferably the front waist region 36, is positioned across the front of the wearer. The diaperer then wraps the side panels 30 around the wearer such that the front waist region 36 and the rear waist region 38 are in an overlapping configuration. The side panels 30 will typically be extended and tensioned during this operation so as to conform to the size and shape of the wearer. The fastening system 40 is secured to effect a side closure.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A diaper (20) comprising:
a) a liquid pervious topsheet (24),
b) a backsheet (26) joined to said topsheet, and
c) a fecal management member (29) positioned between said topsheet (24) and said backsheet,
said fecal management member comprises a support member (120) and a plurality of fibers (118), said fibers being woven into said support member, and said fibers having portions (130) projecting from said support member,
d) an absorbent core (28) positioned between said fecal management member and said backsheet, wherein the support member is selected from the group consisting of a porous film, a breathable film, a nonwoven web, an apertured nonwoven web, strips of nonwoven material, a sheet of loop material, apertured films, apertured formed films, strips of thermoplastic film, a hotmelt material, or strips of hotmelt material, and the support member has a thickness of 0.00125 to 0.05 cm.

2. The diaper (20) of claim 1 where said fiber portions (130) project from only one surface of said support member (120).

3. The diaper (20) according to claim 1 where said fiber portions (130) projecting from said support member (120) are of arcuate form.

4. The diaper (20) according to claim 1 where said fiber portions (130) projecting from said support member (120) are of loop form.

5. The diaper (20) according to claim 1 where said fiber portions (130) projecting from said support member (120) are of pole form.

6. The diaper (20) according to claim 1 wherein said fiber portions (130) projecting from said support member (120) have a height from said support member of at least 0.5 mm.

7. The diaper (20) according to claim 4 wherein said fiber portions (130) projecting from said support member (120) have a height from said support member of at least 1.0 mm.

8. The diaper (20) to claim 1 wherein said fecal management member (29) has a resiliency of at least 85% after 30 seconds under an applied load of 981N/cm².

9. The diaper (20) according to claim 1 wherein said fecal management member (29) has a Compression Resistance of at least 30% under an applied load of 981N/cm².

10. The diaper (20) according to claim 1 wherein said fecal management member (29) has a basis weight between 20 grams per square meter and 500 grams per square meter.

11. The diaper (20) according to claim 1 further comprising a second support member joined to said support member (120) opposite said plurality of fibers (118).

12. The diaper (20) according to claim 1 wherein said fibers (118) have a hydrophilicity which is less than the hydrophilicity of said support member (120).

13. The diaper (20) according to claim 1 wherein said fecal management member (29) is secured to said absorbent core (28) with an adhesive having a hydrophilicity which is greater than the hydrophilicity of said sheet of fibers (118).

14. The diaper (20) according to claim 1 wherein said fecal management member (29) is not secured to said topsheet (24).

15. The diaper (20) according to claim 1 wherein said diaper has a rear waist region (38) and a front waist region (36), said fecal management member (29) being positioned in at least said rear waist region of said disposable absorbent article.

16. The diaper (20) according to claim 1 where said fecal management member (29) has at least a first region and a second region, said fiber portions (130) projecting from said support member (120) in said first region having a height from said support member which is less than the height of said fiber portions projecting from said support member in said second region.

## Patentansprüche

1. Windel (20) mit:
(a) einer flüssigkeitsdurchlässigen Oberschicht (24),
(b) einer Unterschicht (26), die mit der Oberschicht verbunden ist, und
(c) einer Stuhlgang-Managementeinrichtung (29), das zwischen der Oberschicht (24) und der Unterschicht positioniert ist,
wobei die Stuhlgang-Managementeinrichtung (29) ein Stützelement (120) und eine Mehrzahl von Fasern (118) umfaßt; wobei die Fasern in ein Stützelement eingewoben sind, und wobei die Fasern Bereiche (130) haben, die von dem Stützelement vorstehen,
(d) einen absorbierenden Kern (28), der zwischen der Stuhlgang-Managementeinrichtung und der Unterschicht positioniert ist, wobei das Stützelement ausgewählt ist aus der Gruppe bestehend aus einem porösen Film, einem atmungsfähigen Film, einer Vliesbahn, einer geöffneten Vliesbahn, Streifen eines Vliesmaterials, einem Flächengebilde eines Schlingenmaterials, geöffnete Filme, geöffnete, geformte Filme, Streifen eines thermoplastischen Films, ein heiß schmelzendes Material oder Streifen eines heiß schmelzenden Materials, und das Stützelement eine Dicke von 0,00125 bis 0,,05 cm hat.

2. Windel (20) nach Anspruch 1, in welcher die Faserbereiche (130) von nur einer Oberfläche des Stützelements (120) vorstehen.

3. Windel (20) nach Anspruch 1, in welcher die Faserbereiche (130), die von dem Stützelement (120) vorstehen, bogenförmig sind.

4. Windel (20) nach Anspruch 1, in welcher die Faserbereiche (130), die von dem Stützelement (120) vorstehen, schlingenförmig sind.

5. Windel (20) nach Anspruch 1, in welcher die Faserbereiche (130), die von dem Stützelement (120) vorstehen, polförmig sind.

6. Windel (20) nach Anspruch 1, in welcher die Faserbereiche (130), die von dem Stützelement (120) vorstehen, eine Höhe von dem Stützelement von wenigstens 0,5 mm haben.

7. Windel (20) nach Anspruch 4, in welcher die Faserbereiche (130), die von dem Stützelement (120) vorstehen, eine Höhe von dem Stützelement von wenigstens 1,0 mm haben.

8. Windel (20) nach Anspruch 1, in welcher die Stuhlgang-Managementeinrichtung (29) eine Elastizität von wenigstens 85% nach 30 Sekunden unter einer beaufschlagten Last von 981N/cm² hat.

9. Windel (20) nach Anspruch 1, in welcher die Stuhlgang-Managementeinrichtung (29) einen Komprimierungswiderstand von wenigstens 30% und einer beaufschlagten Last von 981N/cm² hat.

10. Windel (20) nach Anspruch 1, in welcher die Stuhlgang-Managementeinrichtung (29) ein Basisgewicht zwischen 20 Gramm pro Quadratmeter und 500 Gramm pro Quadratmeter hat.

11. Windel (20) nach Anspruch 1, ferner mit einem zweiten Stützelement, das mit dem Stützelement (120) entgegen gesetzt zu der Mehrzahl von Fasern (118) verbunden ist.

12. Windel (20) nach Anspruch 1, in welcher die Fasern (118) eine Hydrophilizität haben, welche geringer ist als die Hydrophilizität des Stützelements (120).

13. Windel (20) nach Anspruch 1, in welcher die Stuhlgang-Managementeinrichtung (29) an dem absorbierenden Kern (28) mit einem Haftmittel festgelegt ist, das eine Hydrophilizität aufweist, die größer ist als die Hydrophilizität des Flächengebildes von Fasern (118).

14. Windel (20) nach Anspruch 1, in welcher die Stuhlgang-Managementeinrichtung (29) an der Oberschicht (24) festgelegt ist.

15. Windel (20) nach Anspruch 1, in welcher die Windel eine hintere Taillenregion (38) und eine vordere Taillenregion (36) hat, wobei die Stuhlgang-Managementeinrichtung (29) in wenigstens der hinteren Taillenregion des absorbierenden Einwegartikels positioniert ist.

16. Windel (20) nach Anspruch 1, in welcher die Stuhlgang-Managementeinrichtung (29) wenigstens eine erste Region und eine zweite Region hat, wobei die Faserbereiche (130), die von dem Stützelement (120) in der ersten Region vorstehen, eine Höhe von dem Stützelement haben, welche geringer ist als die Höhe der Faserbereiche, die von dem Stützelement in der zweiten Region vorstehen.

## Revendications

1. Couche (20) comprenant :
a) une feuille de dessus (24) perméable aux liquides,
b) une feuille de fond (26) réunie à ladite feuille de dessus, et
c) un élément (29) de gestion de matières fécales, placé entre ladite feuille de dessus (24) et ladite feuille de fond, ledit élément de gestion de matières fécales comprenant un élément-support (120) et une pluralité de fibres (118), lesdites fibres étant tissées dans ledit élément-support, et lesdites fibres présentant des parties (130) qui font saillie à partir dudit élément-support,
d) une âme absorbante (28) placée entre ledit élément de gestion de matières fécales et ladite feuille de fond,
dans laquelle l'élément-support est choisi dans le groupe constitué par un film poreux, un film pouvant respirer, une nappe non tissée, une nappe non tissée perforée, des bandes de matériau non tissé, une feuille de matériau de boucle, des films perforés, des films formés perforés, des bandes de film thermoplastique, un matériau thermofusible ou des bandes de matériau thermofusible, et l'élément de support a une épaisseur de 0,00125 à 0,05 cm.

2. Couche (20) selon la revendication 1, dans laquelle lesdites parties de fibres (130) font saillie à partir d'une seule surface dudit élément-support (120).

3. Couche (20) selon la revendication 1, dans laquelle lesdites parties de fibres (130) qui font saillie à partir dudit élément-support (120) ont une forme incurvée.

4. Couche (20) selon la revendication 1, dans laquelle lesdites parties de fibres (130) qui font saillie à partir dudit élément-support (120) ont une forme de boucle.

5. Couche (20) selon la revendication 1, dans laquelle lesdites parties de fibres (130) qui font saillie à partir dudit élément-support (120) ont une forme de pôle.

6. Couche (20) selon la revendication 1, dans laquelle lesdites parties de fibres (130) qui font saillie à partir de l'élément-support (120) ont une hauteur par rapport audit élément-support d'au moins 0,5 mm.

7. Couche (20) selon la revendication 4, dans laquelle lesdites parties de fibres (130) qui font saillie à partir dudit élément-support (120) ont une hauteur par rapport audit élément de support d'au moins 1,0 mm.

8. Couche (20) selon la revendication 1, dans laquelle ledit élément (29) de gestion de matières fécales a une résilience d'au moins 85% après 30 secondes sous une charge appliquée de 981 N/cm².

9. Couche (20) selon la revendication 1, dans laquelle ledit élément (29) de gestion de matières fécales a une résistance à la compression d'au moins 30% sous une charge appliquée de 981 N/cm².

10. Couche (20) selon la revendication 1, dans laquelle ledit élément (29) de gestion de matières fécales a un grammage compris entre 20 g/m² et 500 g/m².

11. Couche (20) selon la revendication 1, comprenant, en outre, un deuxième élément-support réuni audit élément-support (120) opposé à ladite pluralité de fibres (118).

12. Couche (20) selon la revendication 1, dans laquelle lesdites fibres (118) ont une hydrophilie qui est inférieure à celle dudit élément-support (120).

13. Couche (20) selon la revendication 1, dans laquelle ledit élément (29) de gestion de matières fécales est assujetti à ladite âme absorbante (28) avec un adhésif ayant une hydrophilie qui est supérieure à celle de ladite feuille de fibres (118).

14. Couche (20) selon la revendication 1, dans laquelle ledit élément (29) de gestion de matières fécales (29) n'est pas assujetti à ladite feuille de dessus (24).

15. Couche (20) selon la revendication 1, dans laquelle ladite couche présente une région de ceinture arrière (38) et une région de ceinture avant (36), ledit élément (29) de gestion de matières fécales étant placé au moins dans ladite région de ceinture arrière dudit article absorbant jetable.

16. Couche (20) selon la revendication 1, dans laquelle ledit élément (29) de gestion de matières fécales présente au moins une première région et une deuxième région, lesdites parties de fibres (130) qui font saillie à partir dudit élément-support (120) dans ladite première région ayant une hauteur par rapport audit élément-support qui est inférieure à celle desdites parties de fibres qui font saillie à partir dudit élément-support dans ladite deuxième région.
